(19) Europäisches Patentamt
European Patent Office
Office européen des brevets

(11) **EP 4 593 035 A1**

(12) **EUROPEAN PATENT APPLICATION**

(43) Date of publication:
**30.07.2025 Bulletin 2025/31**

(21) Application number: **24315027.3**

(22) Date of filing: **24.01.2024**

(51) International Patent Classification (IPC):
**G16H 50/20** (2018.01)          **G06N 3/00** (2023.01)
**G16H 50/30** (2018.01)

(52) Cooperative Patent Classification (CPC):
**G16H 50/20; G06N 3/00; G16H 50/30;** G16H 10/20;
G16H 10/60

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Sanofi**
**75017 Paris (FR)**

(72) Inventors:
• **CHOW, Ohn**
**Cambridge, 02141 (US)**
• **DORMONT, Flavio**
**Cambridge, 02141 (US)**

• **KRIUKOV, Maksim**
**08016 Barcelona (ES)**
• **MORIARTY, Kathleen**
**Toronto, M2R 3T4 (CA)**
• **RUFINO, Brandon**
**Toronto, M2R 3T4 (CA)**
• **MATHUR, Sachin**
**Cambridge, 02141 (US)**

(74) Representative: **Kennedy, Richard E. et al**
**Venner Shipley LLP**
**5 Stirling House**
**Stirling Road**
**The Surrey Research Park**
**Guildford GU2 7RF (GB)**

(54) **PREDICTING DISEASE SEVERITY**

(57)      A computer-implemented method of predicting disease severity in a subject comprises: receiving, at a generalized additive model, input data corresponding to a set of one or more features for the subject, and generating, as an output of the generalized additive model, a predicted disease severity score for the subject, wherein generating the predicted disease severity score for the subject comprises processing the received input data using the generalized additive model.

**EP 4 593 035 A1**

## Description

### Field

This specification relates to a computer-implemented method of predicting disease severity in a subject. It also relates to a computing system for performing the method, and a non-transitory storage medium comprising instructions to perform the method.

### Background

Scores assessing disease severity are defined for many major chronic diseases and these are widely used in randomized clinical trials. However, in real-world settings, such scores may not be consistently calculated by physicians, leading to varying and sometimes limited availability of this information in real world data. Hence, disease severity measures are generally available for only a small subset of patients in real world data.

Rheumatoid arthritis has an estimated global prevalence of 0.46%. DAS-28 is a measure of rheumatoid arthritis disease activity which is widely used in research and, to a lesser extent, in real world clinical practice. DAS stands for "disease activity score" and the number 28 refers to the number of joints which are examined during a clinical assessment used in determining the score.

Atopic dermatitis has an estimated global prevalence of 2.4%. EASI is a measure of atopic dermatitis disease activity which is widely used in research and, to a lesser extent, in real world clinical practice. EASI stands for "eczema area and severity index", and its calculation involves evaluation of four key acute and chronic signs of inflammation (erythema, induration, excoriation and lichenification).

Ulcerative colitis has an estimated global prevalence of 0.16% to 0.29%. The Mayo score is a measure of ulcerative colitis disease activity which is widely used to monitor ulcerative colitis. The Mayo score constitutes of four parts (rectal bleeding, stool frequency, physician assessment, endoscopy appearance) and each part is calculated individually and summed to provide an overall perspective of ulcerative colitis activity.

Alternative means of assessing disease severity scores would be of assistance to clinicians and researchers, for example in understanding the impact of interventions on major chronic diseases in real-world settings.

### Summary

The present specification provides a computer-implemented method of predicting disease severity in a subject. The method comprises receiving, at a generalized additive model, input data corresponding to a set of one or more features for the subject, and generating, as an output of the generalized additive model, a predicted disease severity score for the subject, wherein generating the predicted disease severity score for the subject comprises processing the received input data using the generalized additive model.

The generalized additive model may comprise an explainable boosting machine.

The disease severity score may be a disease severity score for an inflammatory condition.

The set of one or more features may comprise one or more physiological measurements.

The disease severity score may comprise a rheumatoid arthritis disease activity score such as a DAS-28 score.

The set of one or more features may comprise at least one of an erythrocyte sedimentation rate, ESR, measurement for the subject, a C-reactive protein, CRP, measurement for the subject, or a hematocrit measurement for the subject.

In some embodiments the set of one or more features includes an erythrocyte sedimentation rate, ESR, measurement for the subject and a C-reactive protein, CRP, measurement for the subject.

The set of one or more features may comprise at least one of a low-density lipoprotein, LDL, measurement for the subject, a phosphates measurement for the subject, a lactate dehydrogenase, LDH, measurement for the subject, a cholesterol (CHOL) measurement for the subject, a high-density lipoprotein, HDL, measurement for the subject, a blood urea nitrogen, BUN, measurement for the subject, an indirect bilirubin, IB, measurement for the subject, or a urate measurement for the subject.

The disease severity score may be a severity score for atopic dermatitis, such as an EASI score.

The set of one or more features may comprise at least one of: a lactase dehydrogenase, LDH, measurement, for the subject; a C-reactive protein, CRP, measurement for the subject; an allergen-specific immunoglobulin, ALTIGE, measurement for the subject; an eosinophils, EOS, measurement for the subject; a lymphocytes, LYM, measurement for the subject; a creatine kinase, CK, measurement for the subject; a basophils, BASO, measurement for the subject; an aspartate transferase, AST, measurement for the subject, or an albumin, ALB, measurement for the subject.

The set of one or more features may comprises: LDH and CRP, LDH and ALTIGE, ALTIGE and EOS, or EOS and LYM. -

The disease severity score may be a severity score for inflammatory bowel disease, such as a severity score for ulcerative colitis such as a MAYO score.

The set of one or more features may comprise at least one of:

a blood urea nitrogen measurement for the subject;
a hemoglobin measurement for the subject;
a platelets measurement for the subject;
an albumin measurement for the subject;
an alkaline phosphatase measurement for the subject;
a calcium measurement for the subject;
a chloride measurement for the subject;
a creatinine measurement for the subject;
a direct bilirubin measurement for the subject;
a glucose measurement for the subject;
a hematocrit measurement for the subject;
a potassium measurement for the subject;
a red blood cells measurement for the subject;
a sodium measurement for the subject;
a white blood cells measurement for the subject;
a urine red blood cells measurement for the subject, or
a urine white blood cells measurement for the subject.

The set of one or more features may comprise at least two, or all, of:

a blood urea nitrogen measurement for the subject;
a hemoglobin measurement for the subject;
a platelets measurement for the subject;
an albumin measurement for the subject;
an alkaline phosphatase measurement for the subject;
a calcium measurement for the subject;
a chloride measurement for the subject;
a creatinine measurement for the subject;
a direct bilirubin measurement for the subject;
a glucose measurement for the subject;
a hematocrit measurement for the subject;
a potassium measurement for the subject;
a red blood cells measurement for the subject;
a sodium measurement for the subject;
a white blood cells measurement for the subject;
a urine red blood cells measurement for the subject, or
a urine white blood cells measurement for the subject.

The method may further comprise augmenting a patient data set using the generalized additive model, wherein augmenting the patient data set comprises deploying the generalized additive model on a patient data set to predict, for each of a plurality of patients, a predicted disease severity score for the patient. The patient data set may be a set of real world data.

This specification also provides a computer-implemented method of determining a response of a subject to a drug over time, comprising, for each of a plurality of times in a time series, generating a disease severity score for the subject, wherein the disease severity score for the subject at each time is generated based on values of at least one of said set of one or more features at that time.

This specification also provides a computer-implemented method of providing a trained generalized additive model to predict a disease severity score for a subject, the method fitting the generalized additive model to a data set comprising a plurality of data items for a respective plurality of subjects, each data item comprising a disease severity score for the subject and data corresponding to a set of one or more features for the subject.

The trained generalized additive model may comprise an explainable boosting machine.

This specification also provides a non-transitory computer-readable medium comprising instructions, which when executed by a processor, cause the processor to perform a method as described herein.

This specification also provides a computing system comprising one or more processors and one or more memories storing computer readable instructions, which when executed by the one or more processors, cause a method as described herein to be performed.

This specification also provides a method of treating an immune-mediated disease in a subject in need thereof, comprising:

- predicting a disease severity score for the subject, that includes:

  receiving, at a generalized additive model, input data corresponding to a set of one or more features for the subject, and
  generating, as an output of the generalized additive model, the predicted disease severity score for the subject by processing the received input data using the generalized additive model, and

- administering an active substance based on the predicted disease severity score.

This specification also provides a method of treating an immune-mediated disease in a subject in need thereof, comprising administering an active substance to a subject, wherein the subject has been diagnosed as being affected with the immune-mediated disease based on a predicted disease score, the predicted disease score being obtained by:

  receiving, at a generalized additive model, input data corresponding to a set of one or more features for the subject, and
  generating, as an output of the generalized additive model, the predicted disease severity score for the subject by processing the received input data using the generalized additive model.

As used herein, the term "immune-mediated disease" refers to a disease or condition resulting from an abnormal activity of immune cells, such as, e.g., an immune reaction to self-antigens or an aberrant inflammatory response. In some embodiments, the "immune-mediated disease" is an inflammatory disease. Non-limiting examples of inflammatory diseases include atopic dermatitis, rheumatoid arthritis, and juvenile idiopathic arthritis.

In some embodiments, the active substance may be an anti-IL-6 receptor (IL-6R) antibody or an anti-IL-4 receptor (IL-4R) antibody. In some embodiments, the active substance is not limited to an anti-IL-6 receptor (IL-6R) antibody or to an anti-IL-4 receptor (IL-4R) antibody but is an active substance useful to treat the immune-mediated disease. In some embodiments, the active substance is useful to treat atopic dermatitis. In some embodiments, the active substance is useful to rheumatoid arthritis. In some embodiments, the active substance is useful to treat juvenile idiopathic arthritis.

In some embodiments, the immune-mediated disease is atopic dermatitis. In some embodiments, the immune-mediated disease is atopic dermatitis and the active substance is an anti-IL-4 receptor (IL-4R) antibody. In some embodiments, the immune-mediated disease is rheumatoid arthritis. In some embodiments, the immune-mediated disease is rheumatoid arthritis and the active substance is an anti-IL-6 receptor (IL-6R) antibody. In some embodiments, the immune-mediated disease is juvenile idiopathic arthritis. In some embodiments, the immune-mediated disease is juvenile idiopathic arthritis and the active substance is an anti-IL-6 receptor (IL-6R) antibody.

This specification also provides a method of treating atopic dermatitis in a subject in need thereof, comprising:

- predicting a disease severity score for the subject, that includes:

  ◦ receiving, at a generalized additive model, input data corresponding to a set of one or more features for the subject, and
  ◦ generating, as an output of the generalized additive model, the predicted disease severity score for the subject by processing the received input data using the generalized additive model, and

- administering an active substance to the subject. In some embodiments, the active substance comprises an anti-interleukin-4-receptor (IL-4R) antibody or an antigen-binding fragment. In some embodiments, the active substance (e.g., the anti-IL-4R antibody or antigen binding fragment thereof) is administered in a dosing regimen based on the predicted disease severity score.

The dosing regimen may comprise an initial dose followed by one or more secondary doses, wherein the initial dose is 600 mg of the active substance (e.g., the anti-IL-4R antibody or antigen-binding fragment thereof), the secondary dose is 300 mg of the active substance (e.g., the anti-IL-4R antibody or antigen-binding fragment thereof), and each secondary dose is administered 1 to 2 weeks after the immediately preceding dose.

This specification also provides a method of treating rheumatoid arthritis in a subject in need thereof, comprising:

- predicting a disease severity score for the subject, that includes:

    ◦ receiving, at a generalized additive model, input data corresponding to a set of one or more features for the subject, and
    ◦ generating, as an output of the generalized additive model, the predicted disease severity score for the subject by processing the received input data using the generalized additive model; and

- administering an active substance to the patient. In some embodiments, the active substance comprises an anti-interleukin-6-receptor (IL-6R) antibody or an antigen-binding fragment thereof. In some embodiments, the active substance (e.g., the anti-IL-6R antibody or antigen binding fragment thereof) is administered in a dosing regimen based on the predicted disease severity score.

The dosing regimen may comprise one or more doses of from about 150 mg to about 200 mg of the active substance (e.g., the anti-IL-6R antibody or antigen-binding fragment thereof) administered once every two weeks.

This specification also provides a method of treating juvenile idiopathic arthritis (JIA), preferably polyarticular-course JIA (pcJIA) or systemic JIA (sJIA), more preferably pcJIA, in a subject in need thereof, comprising:

- predicting a disease severity score for the subject, that includes:

    ◦ receiving, at a generalized additive model, input data corresponding to a set of one or more features for the subject, and
    ◦ generating, as an output of the generalized additive model, the predicted disease severity score for the subject by processing the received input data using the generalized additive model; and

- administering an active substance to the subject. In some embodiments, the active substance comprises an anti-interleukin-6-receptor (IL-6R) antibody or an antigen-binding fragment thereof. In some embodiments, the active substance (e.g., the anti-IL-6R antibody or antigen binding fragment thereof) is administered in a dosing regimen based on the predicted disease severity score.

The dosing regimen may comprise one or more doses of from about 2 mg/kg to about 4 mg/kg of the active substance (e.g., the anti-IL-6R antibody or antigen-binding fragment thereof) administered once every two weeks.

The subject's body weight may be greater than or equal to 10 kg and less than 30 kg and the dosing regimen may comprise one or more doses of about 4 mg/kg of the active substance (e.g.,the anti-IL-6R antibody or antigen-binding fragment thereof) administered once every two weeks.

The subject's body weight may be greater than or equal to 30 kg and the dosing regimen may comprise one or more doses of about 3 mg/kg of the active substance (e.g., the anti-IL-6R antibody or antigen-binding fragment thereof) administered once every two weeks, and each one or more doses of the active substance (e.g., the anti-IL-6R antibody or antigen-binding fragment thereof) is capped at 200 mg.

This specification also provides an active substance, for use in a method of treating an immune-mediated disease in a subject in need thereof, wherein the method of treating the subject comprises

- predicting a disease severity score for the subject, that includes:

    ◦ receiving, at a generalized additive model, input data corresponding to a set of one or more features for the subject, and
    ◦ generating, as an output of the generalized additive model, the predicted disease severity score for the subject by processing the received input data using the generalized additive model, and

- administering the active substance based on the predicted disease severity score.

In some embodiments, the active substance may be an anti-IL-6 receptor (IL-6R) antibody or an anti-IL-4 receptor (IL-4R) antibody. In some embodiments, the active substance is not limited to an anti-IL-6 receptor (IL-6R) antibody or to an anti-IL-4 receptor (IL-4R) antibody but is an active substance useful to treat the immune-mediated disease. In some embodiments, the active substance is useful to treat atopic dermatitis. In some embodiments, the active substance is useful to rheumatoid arthritis. In some embodiments, the active substance is useful to treat juvenile idiopathic arthritis.

In some embodiments, the immune-mediated disease is atopic dermatitis. In some embodiments, the immune-mediated disease is atopic dermatitis and the active substance is an anti-IL-4 receptor (IL-4R) antibody. In some embodiments, the immune-mediated disease is rheumatoid arthritis. In some embodiments, the immune-mediated disease is rheumatoid arthritis and the active substance is an anti-IL-6 receptor (IL-6R) antibody. In some embodiments, the immune-mediated disease is juvenile idiopathic arthritis. In some embodiments, the immune-mediated disease is juvenile idiopathic arthritis and the active substance is an anti-IL-6 receptor (IL-6R) antibody.

This specification also provides an active substance for use in a method of treating atopic dermatitis in a subject in need thereof, wherein the method of treating the subject comprises:

- predicting a disease severity score for the subject, that includes:

  ∘ receiving, at a generalized additive model, input data corresponding to a set of one or more features for the subject, and
  ∘ generating, as an output of the generalized additive model, the predicted disease severity score for the subject by processing the received input data using the generalized additive model; and

- administering the active substance to the subject in a dosing regimen based on the predicted disease severity score.

In some embodiments, the active substance comprises an anti-IL-4R antibody or antigen-binding fragment thereof.

The dosing regimen may comprise an initial dose followed by one or more secondary doses, wherein the initial dose is 600 mg of the active substance (e.g., the anti-IL-4R antibody or antigen-binding fragment thereof), the secondary dose is 300 mg of the active substance (e.g., the anti-IL-4R antibody or antigen-binding fragment thereof), and each secondary dose is administered 1 to 2 weeks after the immediately preceding dose.

This specification also provides an active substance for use in a method of treating rheumatoid arthritis in a subject in need thereof, wherein the method of treating the subject comprises:

- predicting a disease severity score for the subject, that includes:

  ∘ receiving, at a generalized additive model, input data corresponding to a set of one or more features for the subject, and
  ∘ generating, as an output of the generalized additive model, the predicted disease severity score for the subject by processing the received input data using the generalized additive model; and

- administering the active substance to the subject in a dosing regimen based on the predicted disease severity score.

In some embodiments, the active substance comprises an anti-interleukin-6-receptor (IL-6R) antibody or an antigen-binding fragment thereof.

The dosing regimen may comprise one or more doses of from about 150 mg to about 200 mg of the active substance (e.g., the anti-IL-6R antibody or antigen-binding fragment thereof) to be administered once every two weeks.

This specification also provides an active substance for use in a method of treating juvenile idiopathic arthritis (JIA), preferably polyarticular-course JIA (pcJIA) or systemic JIA (sJIA), more preferably pcJIA, in a subject in need thereof, wherein the method of treating the subject comprises:

- predicting a disease severity score for the subject, that includes:

  ∘ receiving, at a generalized additive model, input data corresponding to a set of one or more features for the subject, and
  ∘ generating, as an output of the generalized additive model, the predicted disease severity score for the subject by processing the received input data using the generalized additive model, and

- administering the active substance (e.g., the anti-IL-6R antibody or an antigen-binding fragment thereof to the subject in a dosing regimen based on the predicted disease severity score).

In some embodiments, the active substance comprises an anti-interleukin-6-receptor (IL-6R) antibody or an antigen-binding fragment thereof.

The dosing regimen may comprise one or more doses of from about 2 mg/kg to about 4 mg/kg of the active substance (e.g., the anti-IL-6R antibody or antigen-binding fragment thereof) to be administered once every two weeks.

The subject's body weight may be greater than or equal to 10 kg and less than 30 kg and the dosing regimen comprises one or more doses of about 4 mg/kg of the active substance (e.g., the anti-IL-6R antibody or antigen-binding fragment thereof) to be administered once every two weeks.

The subject's body weight may be greater than or equal to 30 kg and the dosing regimen comprises one or more doses of about 3 mg/kg of the active substance (e.g., the anti-IL-6R antibody or antigen-binding fragment thereof) to be administered once every two weeks, and each one or more doses of the active substance (e.g., the anti-IL-6R antibody or antigen-binding fragment thereof) is capped at 200 mg.

**Brief Description of the Drawings**

So that the invention may be more easily understood, examples thereof will now be described with reference to the accompanying figures, in which:

Figure 1 illustrates a method of augmenting a patient data set to include proxy endpoint data in accordance with an example implementation;
Figure 2 illustrates a feature selection pipeline in accordance with an example implementation;
Figure 3 illustrates use of a trained model to predict a value for an endpoint in accordance with an example implementation;
Figure 4 is a flow diagram of an example process whereby an explainable boosting machine is used to predict a disease severity score for a subject, and
Figure 5 is a schematic of an example system/apparatus which may be used for performing methods described herein.

**Detailed Description**

Overview

A Randomised Control Trial (RCT) is an experimental approach which may be used to evaluate a treatment's clinical efficacy. RCT participants are randomly allocated into one or more treatment and control arms and outcomes of these arms are then directly compared. Participant randomisation strengthens the validity of causal inference by ensuring that each arm as free from bias possible on both observed and unobserved characteristics. Data from RCTs (herein referred to as "RCT data") is captured in tightly controlled settings and is deemed to be of the highest reliability.

Another type of data is real world data (RWD), which is captured outside the context of RCTs through analysis of the data from the healthcare setting. While RCTs test specific hypothesis on efficacy and safety of new drugs in a highly controlled setting, RWD helps with understanding the interaction between patient and treatment in routine clinical practice. RWD and RCT data are therefore often complementary. Real World Evidence (RWE) studies, which are based on RWD, commonly do not yield absolute causal inference due to variability in numerous confounders, but their large sample sizes can offer new insights. However, in order to use RWD for drug discovery studies on millions of individuals, it would be necessary for the RWD to include a disease severity score (i.e. an endpoint) for these individuals; this is not usually available.

The present specification describes an advanced analytics pipeline for RCT signal amplification, which uses RCT data to create a model for a proxy endpoint (e.g. a disease severity score) from limited features available in the RWD data. Using such a proxy endpoint, RWD can be leveraged to study larger cohorts of patients when analysing treatment-effects in routine clinical practice, effectively enabling the integration of RWD and RCT data for predicting patient responses.

As discussed in more detail below, the present specification describes use of Generalized Additive Models (GAMs), and more specifically, Explainable Boosting Machines (EBMs), as the model(s) for predicting a proxy endpoint. Advantageously, GAMs maintain the strong interpretability properties of classical tools such as logistic and linear regression, whilst allowing for discovery of nonlinear effects in a data-driven fashion. More particularly, using EBMs, each feature can be trained independently with boosting to mitigate the effects of co-linearity and learn the best corresponding feature function. As a result, EBMs advantageously maintain both high accuracy and intelligibility and showing comparable performance to other state of the art machine learning methods.

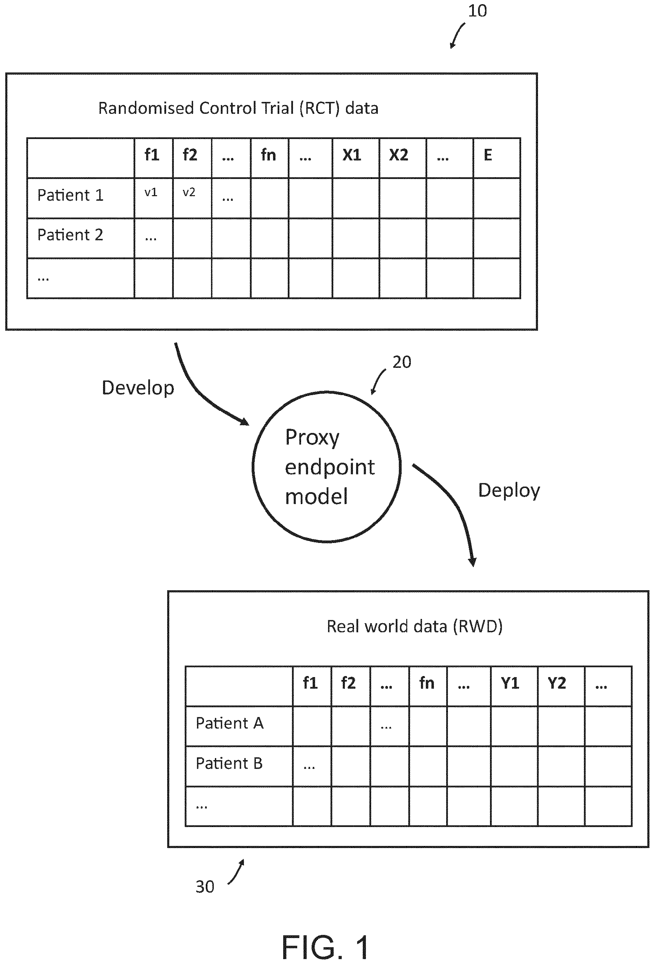

Figure 1 illustrates a method of augmenting a patient data set to include proxy endpoint data in accordance with an example embodiment. As shown, a randomised control trial (RCT) data set 10 is used to develop a proxy endpoint model 20, which in turn is deployed on a patient data set in the form of real world data (RWD) set 30.

As shown, the RCT data set 10 includes data for a number of subjects (e.g. patients) such as records for "Patient 1", "Patient 2" etc as indicated in Figure 1. Similarly, the RWD set 30 includes data for a number of subjects (e.g. patients) such as records for "Patient A", "Patient B" etc as indicated in Figure 1.

As illustrated in Figure 1, the RCT data set 10 includes values (e.g. v1, v2) for particular patient-level features of the data set (e.g. f1, f2 ... fn, ... X1, X2). A wide variety of features may be included depending on the trial. By way of example, features may for example include lab test data such as physiological measurements (e.g. blood test measurements), demographic information such as gender and age, as well as information on medicaments taken, health events, and other biomedical factors.

The RCT data also includes values for a clinical trial endpoint (E). A clinical trial "endpoint" refers to an event or outcome which can be measured objectively to determine whether the intervention being studied is beneficial. For example, an endpoint may comprise a measure of disease severity, for example a disease severity score for a major chronic disease.

The endpoint may comprise a disease severity score for an inflammatory condition. For example, the endpoint may comprise a disease severity score for rheumatoid arthritis (RA) such as a Disease Activity Score 28 (DAS28), which is a measure of severity that is calculated by examining 28 joints for swelling and tenderness. The DAS28 scoring system provides a score between 0 and 10, a larger number indicating more active disease. A subject with a DAS28 score of less than 2.6 is considered as not affected with RA, or in remission from RA; a score greater than or equal to 2.6 and less than 3.2 indicates low activity RA; a score greater than or equal to 3.2 and less than 5.1 indicates moderate activity RA; and a score of 5.1 or greater indicates high activity RA. The DAS28 score may comprise a DAS28-CRP score or a DAS28-ESR score, which combines the DAS28 score with a laboratory data, specifically C-reactive protein levels or erythrocyte sedimentation rate, respectively. Thresholds for DAS28-CRP scores are <2.5 for subjects not affected with RA, or in remission from RA; between 2.5 and 2.9 for low activity RA; between 2.9 and 4.6 for moderate activity RA; and above 4.6 for high activity RA. For DAS28-ESR, thresholds are <2.6 for subjects not affected with RA, or in remission from RA; between 2.6 and 3.2 for low activity RA; between 3.2 and 5.1 for moderate activity RA; and above 5.1 for high activity RA.

In another example, the endpoint may be a disease severity score for atopic dermatitis (AD) such as the Eczema Areas and Severity Index (EASI), which is a composite score integrating body surface (i.e., area of involvement visually estimated in each of the 4 body regions separately - head and neck, upper extremities, trunk, and lower extremities) and intensity of lesional skin in each of the 4 body region for 4 signs (erythema, edema/papulation, excoriation and lichenification). The final EASI score is the summation of the 4 regional scores after normalization by an adult (>8 years old) or pediatric multiplier that reflects the relative contribution of that region to the total body surface area. The EASI score ranges from 0 to 72, with a score of 0 indicating no affection; a score of 0.1 to 1.0 indicating almost clear AD; from 1.1 to 7 for mild AD; from 7.1 to 21 for moderate AD; from 21.1 to 50 for severe AD; and greater than 51 for very severe AD.

In another example, the endpoint may be a disease severity score for inflammatory bowel disease (IBD), for example a severity score for ulcerative colitis such as a MAYO score. The "full" MAYO score includes an assessment of two patient-reported outcomes (stool frequency and rectal bleeding), the endoscopic appearance of the mucosa (endoscopic score), and a physician's global assessment, each of which are scored on a scale from 0 to 3, giving a maximum total score of 12. A "modified" MAYO score is also commonly used, including all assessments of the full MAYO score but the physician's global assessment, giving a maximum total score of 9. Finally, a "partial" MAYO score includes all assessments of the full MAYO score but the endoscopic score, also giving a maximum total score of 9. Thresholds for the full MAYO score are <3 for subjects not affected with IBD, or in remission from IBD; between 3 and 5 for low activity IBD; between 6 and 10 for moderate activity IBD; and between 11 and 12 for high activity IBD. Thresholds for the modified and partial MAYO score are <2 for subjects not affected with IBD, or in remission from IBD; between 2 and 4 for low activity IBD; between 5 and 7 for moderate activity IBD; and between 7 and 9 for high activity IBD.

Referring again to Figure 1, features f1, f2 ... fn denote certain biomedical features which have been selected as candidates for use in developing a proxy endpoint model which predicts a value of the endpoint based on values of the features. Features X1, X2 ... Xm denote other features present in the RCT data, and features Y1, Y2 ... Ym denote other features present in the RWD.

Feature selection

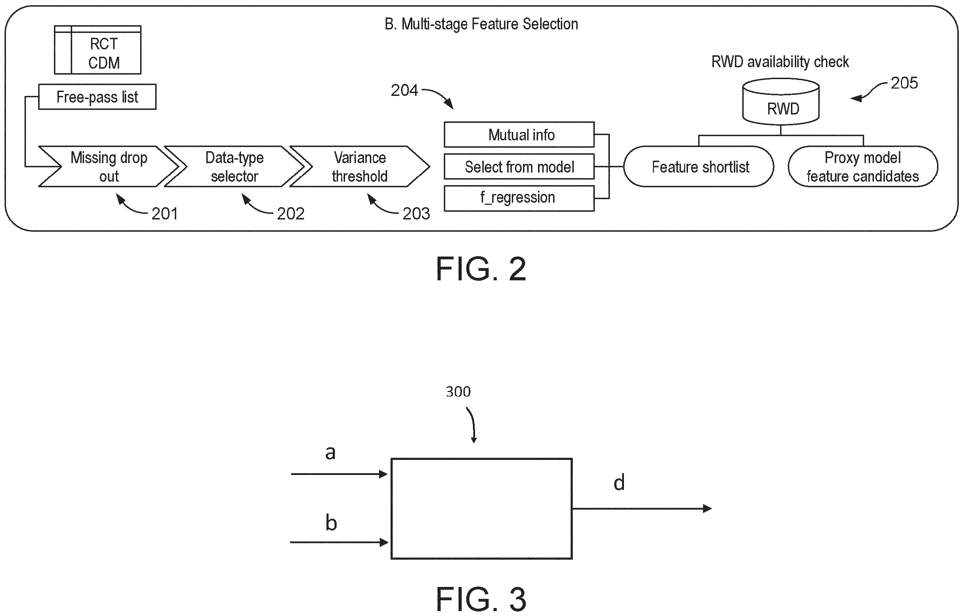

Figure 2 illustrates a feature selection pipeline in accordance with an example implementation. In general, feature selection involves processing an initial set of features in the RCT data in one or more (e.g. multiple) automated selection stages. Each stage receives an input set of features and selects features from the input set if particular criteria are met, thereby to provide an output set of features which may then be used as the input for the next stage. In some cases, a stage may select features by removing other features, e.g. by removing sparse features, non-numerical features, or features which do not change between individuals. The one or more stages may comprise a stage in which features are selected based on their prognostic power to predict the endpoint. Alternatively, or in addition, the one or more stages may comprise a stage in which features are selected based on their availability in the RWD.

Figure 2 illustrates a sequence of automated stages 201-205 which may be applied to the input RCT data in order to automatically select candidate features for use in developing a proxy endpoint model. Although Figure 2 illustrates a particular sequence of stages 201-205, it will be understood that each of the stages 201-205 may also be used as part of other sequences of one or more stages formed from all or a subset of the stages 201-205 and/or other stages.

In stage 201, sparse features are removed. For example, features which are missing from more than 50% of patient feature vectors may be dropped. In the second stage 202, the data is cleaned to remove non-numerical features (e.g study identifier) that do not pertain to predictive biomedical factors. In the third stage 203, features are dropped which do not change between individuals.

In the fourth stage 204, features are ranked in accordance with their prognostic power to predict the endpoint. As shown in Figure 2, this may be done using a number of techniques, labelled "mutual info", "select from model" and "f_regression". It will be understood that each of these techniques may be used individually or in any combination.

"Select from model" refers to the use of shallow tree-based models which are trained and used to rank features according to impurity-based feature importance. XGBoost or Random Forest architectures may be used for this purpose.

"f_regression" refers to use of univariate linear regression used to rank features according to F-statistic.

"mutual info" refers to ranking using custom mutual information adapted for regression tasks.

For each technique, the top 10% of features may be greedily selected and the union of these may be taken as the feature shortlist output for the fourth stage.

In the fifth stage 205, these features are filtered based on their availability in the RWD. For example, if a feature is available in at least 3x more patients than the respective RCT dataset, it may be included in a final list of candidate features. In this way, the method ensures that the final candidate features are present for at least some patients in the RWD and also for at least some patients in the RCT data set.

The candidate features f1, f2, ... fn, may then be evaluated by a subject matter expert to determine one or more sets of model features which are used to train one or more GAM (or more specifically) EBM, models for RWD deployment. In some examples a number of models may be generated with different model features, each model having a different subset of model features selected from the candidate features f1, f2 ... fn.

Training, evaluating and selecting models

An Explainable Boosting Machine (EBM) may be used to develop a proxy endpoint model. An EBM is a Generalized Additive Model (GAM) that learns the target variable using the following function:

$$g(E[y]) = \beta_0 + \sum f_k(x_k)$$

Where g is the link function which adapts the model to fit both continuous and categorical outcomes. In addition, EBMs can be extended to include pairwise and higher order terms, thereby to additionally augment model intelligibility by identifying the combined effect of two or more predictors.

$$g(E[y]) = \beta_0 + \sum f_k(x_k) + \sum f_{kl}(x_k, x_l)$$

Reference is directed to Harsha Nori, Samuel Jenkins, Paul Koch, and Rich Caruana. "Interpretml: A unified framework for machine learning interpretability", arXiv:1909.09223, 2019, which describes a suitable EBM implementation which leverages gradient boosting and bagging to train the model on a set of training data.

A training data set for training the model may be extracted from the RCT data set 10. The training data set at least includes values for the model features and values for the endpoint E. Developing the model 20 comprises fitting the model 20 to the training data set. In this way, a trained model 20 is developed which receives, as input, the values for the model features and generates as output, a corresponding value for the endpoint E. The model may be evaluated on a test set extracted from the RCT data using a performance measure such as MSE, MAE, R2 and/or Spearman correlation between predicted values and actual severity scores. Methods for fitting and testing suitable models such as GAMs and in particular EBMs on data are well known to those skilled in the art and will not be described in detail here.

If the performance measure is above a threshold (e.g. $R2_{test} >= 0.25$, $Spearman_{test} >= 0.5$), the model may be added to a deployable set of models for use in augmenting the RWD set 30 to include proxy endpoint data.

Deployment of a model comprises using the model to predict a value for the RCT endpoint based on the values of the model features in the RWD data set 30, for each patient in the RWD set 30 for which all of the model features were recorded at least once. In general, the model predicts a value for the RCT endpoint at a particular point in time, based on values of the model features in the RWD data set for that time.

It will be understood that the endpoint used in the RCT data set is in general not directly observable in the RWD data set, i.e. the term "RCT endpoint" generally refers to the endpoint observed in the RCT data set, but not in the RWD.

As a soft threshold, model performance may be considered as being acceptable for RWD deployment if the model (1) uses features that are available in RWD, (2) utilizes clinically relevant features and (3) achieves test set performance of $R2_{test} >= 0.25$, $Spearman_{test} >= 0.5$.

Figure 3 illustrates use of a trained EBM model 300 to predict a value for an endpoint (in this case, a disease severity score) based on values a, b of the model features in the RWD data set. As shown, the model 300 processes the inputs a, b and predicts a disease severity score d as output. Although two model features are shown in Figure 3, it will be appreciated that different numbers of model features may be used in appropriate cases. In some examples, the model features comprise one or more physiological measurements such as one or more blood test measurements.

Atopic Dermatitis (AD) Proxy Endpoint Models

A set of proxy models were trained using an atopic dermatitis (AD) RCT data set derived from clinical trial data. The features selection approach described above was applied and four feature subsets were tested as shown in table 1 below. The "Multiple strategy" approach included the full list of candidate features from the automated multi-stage feature selection approach described above in connection with Figure 2 (hereafter "the present feature selection approach"), while the other feature subsets were determined in light of this information but also with assistance of subject matter experts.

Table 1: Comparison of predictive modeling methods using fixed feature sets from multi-stage feature selection on AD dataset. Bolded is the best achieved metric across various methods. Underlined is the best achieved metric within the same feature set group.

| Proxy Model | RWD Availability Rank | Top-10 Features (In decreasing order of importance) | Model Type | R2 Train | R2 Test | Spearman Train | Spearman Test | MAE Train | MAE Test | MSE Train | MSE Test |
|---|---|---|---|---|---|---|---|---|---|---|---|
| Multiple strategy | 4 | LDH, CRP, ALTIGE, EOS, LYM, Age, CK, Antihistamines for systemic use flag, Corticosteroids potent (group IV), BASO | EBM | 0.50 | 0.32 | 0.72 | 0.56 | 0.55 | 0.56 | 0.48 | 0.51 |
| | | | LR | 0.37 | 0.26 | 0.63 | 0.55 | 0.63 | 0.60 | 0.62 | 0.56 |
| | | | RF | 0.93 | 0.32 | 0.98 | 0.57 | 0.29 | 0.57 | 0.07 | 0.52 |
| | | | GBM | 0.56 | 0.37 | 0.76 | 0.59 | 0.51 | 0.54 | 0.42 | 0.48 |
| | | | SVM | 0.36 | 0.27 | 0.64 | 0.57 | 0.63 | 0.60 | 0.62 | 0.55 |
| LDH set | 3 | LDH, ALTIGE, Allergic conjunctivitis (keratoconjunctivitis) flag, LYM, EOS, BASO, CRP, CK, Sex, Age | EBM | 0.45 | 0.38 | 0.69 | 0.61 | 0.58 | 0.53 | 0.53 | 0.47 |
| | | | LR | 0.32 | 0.33 | 0.61 | 0.61 | 0.65 | 0.55 | 0.66 | 0.51 |
| | | | RF | 0.93 | 0.30 | 0.97 | 0.55 | 0.29 | 0.57 | 0.07 | 0.53 |
| | | | GBM | 0.52 | 0.41 | 0.74 | 0.61 | 0.54 | 0.52 | 0.47 | 0.45 |
| | | | SVM | 0.32 | 0.33 | 0.61 | 0.61 | 0.65 | 0.56 | 0.66 | 0.51 |
| IGE set | 2 | ALTIGE, EOS, LYM, Allergic conjunctivitis (keratoconjunctivitis) flag, CRP, BASO, AST, Age, Sex, ALB | EBM | 0.28 | 0.27 | 0.63 | 0.52 | 0.62 | 0.58 | 0.60 | 0.55 |
| | | | LR | 0.24 | 0.18 | 0.52 | 0.48 | 0.69 | 0.62 | 0.74 | 0.62 |
| | | | RF | 0.90 | 0.18 | 0.97 | 0.45 | 0.24 | 0.62 | 0.10 | 0.62 |
| | | | GBM | 0.45 | 0.23 | 0.69 | 0.50 | 0.58 | 0.59 | 0.53 | 0.58 |
| | | | SVM | 0.22 | 0.18 | 0.51 | 0.50 | 0.70 | 0.63 | 0.75 | 0.62 |
| EOS set | 1 | EOS, LYM, Allergic conjunctivitis (keratoconjunctivitis) flag, CRP, AST, Sex, BASO, ALB, Race, Age | EBM | 0.34 | 0.24 | 0.59 | 0.50 | 0.64 | 0.58 | 0.64 | 0.57 |
| | | | LR | 0.21 | 0.14 | 0.49 | 0.43 | 0.70 | 0.63 | 0.76 | 0.65 |
| | | | RF | 0.88 | 0.17 | 0.96 | 0.40 | 0.26 | 0.62 | 0.11 | 0.62 |
| | | | GBM | 0.43 | 0.19 | 0.67 | 0.44 | 0.59 | 0.60 | 0.56 | 0.61 |
| | | | SVM | 0.21 | 0.14 | 0.49 | 0.43 | 0.70 | 0.63 | 0.77 | 0.65 |

Abbreviations: LDH - lactate dehydrogenase, CRP - C-reactive protein, ALTIGE - allergen-specific immunoglobulin E, EOS - eosinophils, LYM - lymphocytes, CK - creatine kinase, BASO - basophils, AST - aspartate transferase, ALB - albumin, EMB - explainable boosting machine, LR - linear regression, RF - random forest, GBM - gradient boosting model, SVM - support vector machine.

Table 2 shows the best performing proxy endpoint model in comparison with other feature selectors and other model types. It can be seen that the present feature selection approach led to the best results overall. When combined with EBM, it was the top performer in terms of spearman correlations. The next best-performing feature selection approach involved the use of Lasso sparse selection with downstream gradient boosting or random forest models ($R_2$ of 0.33 and 0.32, respectively). Other feature selection methods including RFE and AIC led to inferior results.

Table 2: Predictive modeling results for AD dataset. Bold - best R2 and Spearman result. Multi-stage feature selection results depict only the best metric obtained out of the set of proxy models created. Full performance results of other proxy models are shown in supporting results.

| Feature selector | Spearman Test (6)* | | Lasso (38) | | RFE (191) | | AIC (93) | | Multi-stage feature selection (20) | |
|---|---|---|---|---|---|---|---|---|---|---|
| Regressor | Test R2 | Test Spearman | Test R2 | Test Spearman | Test R2 | Test Spearman | Test R2 | Test Spearman | Test R2 | Test Spearman |
| Linear Regression | 0.30 | 0.60 | 0.28 | 0.59 | 0.09 | 0.41 | 0.06 | 0.41 | 0.33 | 0.61 |
| Random Forest | 0.18 | 0.50 | 0.33 | 0.58 | 0.32 | 0.56 | 0.28 | 0.52 | 0.30 | 0.55 |
| Gradient Boosting Regression | 0.32 | 0.55 | 0.32 | 0.54 | 0.19 | 0.47 | 0.28 | 0.51 | 0.41 | 0.61 |
| SVM | 0.30 | 0.60 | 0.29 | 0.60 | 0.24 | 0.50 | 0.22 | 0.49 | 0.33 | 0.61 |
| EBM | 0.38 | 0.60 | 0.27 | 0.53 | 0.18 | 0.45 | 0.17 | 0.44 | 0.38 | 0.61 |
| * The number in parentheses indicates the number of selected features for each method. | | | | | | | | | | |

When evaluating the features selected, LDH, EOS and ALTIGE were found to be the most significant features for accurate prediction of the EASI score. Both the present feature selection approach and the Lasso and Spearman feature selection methods included these in their final feature set. Downstream modeling indicates that these features account for more than 20% of the total predictive power. Indeed, previous studies highlighted the role of ALTIGE and EOS in AD pathogenesis and their potential use for evaluating disease outcomes. LDH has also been associated with AD severity and could be used in combination with other factors for disease monitoring. The present feature selection approach exclusively selected basophils and cholesterol as important biomarkers. Basophils are believed to be involved in type 2 inflammation among patients with AD, while cholesterol levels describe the state of lipid layer. A potential breach in the lipid layer can trigger the onset and progression of AD. Finally, the performance of the present feature selection approach may be attributed to the inclusion of patients' race as a predictor of disease severity. Based on published reports, AD occurs more frequently in non-White population and, particularly, patients with African descent have more severe dermatitis cases possibly associated with FLG mutation.

Rheumatoid Arthritis (RA) Proxy Endpoint Models

The present feature selection approach was applied to a rheumatoid arthritis (RA) dataset derived from clinical trial data. 158 features were input into the first stage 201 and 42 lab tests were dropped due to sparseness. No features were dropped due to data type (stage 202). In the variance threshold stage (stage 203), 1 lab test was dropped and 3 concomitant medication flags. As expected, in the fourth stage 204 using ranking-based selectors, the largest drop in features across all categories was observed. Notably, only one subject-level flag and 4 medical history flags were kept while all concomitant medication information was filtered due to their limited predictive power. In contrast, a total of 34 lab tests (measurements) were retained, primary encompassing hematology tests and RA-specific measurements such as tender and swollen joint counts. It was next checked whether the selected variables have sufficient availability in real world data. Based on the RWD RA cohort, 5 physician assessment measurements were identified that are not well represented in the RWD dataset (CDAI, PGA, TJC28, SJC28 and SGA) and so these were excluded. The remaining 34 features were used as final proxy candidates.

Feature sets for RWD deployment were selected in collaboration with RA subject-matter experts. Four feature subsets were tested as shown in table 3 below.

Table 3: Comparison of predictive modeling methods using fixed feature sets from multi-stage feature selection on RA dataset. Bolded is the best achieved metric across various methods. Underlined is the best achieved metric within the same feature set group.

| Proxy Model | RWD Availability Rank | Top-10 Features (In decreasing order of importance) | Model Type | R2 Train | R2 Test | Spearman Train | Spearman Test | MAE Train | MAE Test | MSE Train | MSE Test |
|---|---|---|---|---|---|---|---|---|---|---|---|
| Multiple strategy | 4 | CRP, ESR, LDL, P, LDH, CHOL, HDL, BUN, IB, UA | EBM | 0.66 | 0.54 | 0.82 | 0.75 | 0.72 | 0.83 | 0.82 | 1.27 |
| | | | LR | 0.47 | 0.39 | 0.67 | 0.67 | 0.96 | 1.05 | 1.39 | 1.68 |
| | | | RF | 0.94 | 0.57 | 0.98 | 0.75 | 0.30 | 0.90 | 0.14 | 1.20 |
| | | | GBM | 0.67 | 0.57 | 0.82 | 0.76 | 0.71 | 0.90 | 0.80 | 1.18 |
| | | | SVM | 0.39 | 0.41 | 0.67 | 0.67 | 0.98 | 1.04 | 1.43 | 1.68 |
| ESR | 1 | ESR | EBM | 0.28 | 0.27 | 0.52 | 0.52 | 1.06 | 1.13 | 1.74 | 2.02 |
| | | | LR | 0.18 | 0.18 | 0.42 | 0.46 | 1.15 | 1.23 | 2.00 | 2.27 |
| | | | RF | 0.30 | 0.27 | 0.54 | 0.52 | 1.04 | 1.13 | 1.69 | 2.01 |
| | | | GBM | 0.30 | 0.28 | 0.53 | 0.53 | 1.04 | 1.12 | 1.70 | 2.00 |
| | | | SVM | 0.18 | 0.18 | 0.42 | 0.46 | 1.15 | 1.23 | 2.00 | 2.27 |
| CRP | 2 | CRP | EBM | 0.39 | 0.43 | 0.61 | 0.65 | 1.00 | 1.06 | 1.48 | 1.58 |
| | | | LR | 0.24 | 0.28 | 0.60 | 0.65 | 1.12 | 1.17 | 1.84 | 2.00 |
| | | | RF | 0.54 | 0.32 | 0.72 | 0.58 | 0.85 | 1.13 | 1.11 | 1.89 |
| | | | GBM | 0.41 | 0.42 | 0.63 | 0.65 | 0.98 | 1.07 | 1.42 | 1.62 |
| | | | SVM | 0.24 | 0.28 | 0.60 | 0.65 | 1.12 | 1.17 | 1.84 | 2.00 |
| ESR & CRP | 3 | ESR, CRP | EBM | 0.47 | 0.48 | 0.68 | 0.69 | 0.90 | 0.97 | 1.28 | 1.43 |
| | | | LR | 0.29 | 0.29 | 0.61 | 0.64 | 1.07 | 1.14 | 1.72 | 1.97 |
| | | | RF | 0.66 | 0.40 | 0.81 | 0.64 | 0.72 | 1.03 | 0.83 | 1.66 |
| | | | GBM | 0.53 | 0.46 | 0.72 | 0.68 | 0.85 | 0.99 | 1.13 | 1.49 |
| | | | SVM | 0.29 | 0.29 | 0.61 | 0.64 | 1.07 | 1.14 | 1.72 | 1.97 |

Abbreviations: CRP - C-reactive protein, ESR - erythrocyte segmentation rate, LDL - low density lipoprotein, P - phosphates, LDH - lactate dehydrogenase, CHOL - cholesterol, HDL - high density lipoprotein, BUN - blood urea nitrogen, IB - indirect bilirubin, UA - urate, EMB - explainable boosting machine, LR - linear regression, RF - random forest, GBM - gradient boosting model, SVM - support vector machine.

The combination of the present feature selection approach ("multiple strategy" mode) and an EBM architecture, achieved the best performance, achieving an R2 score of 0.54 and Spearman score of 0.75 on the held-out test set (see table 4 below). Following discussions with subject-matter experts, three models that only use ESR, CRP, or both were also trained. These models use smaller sets of features making them more flexible for wider application, but with a cost of reduced accuracy.

Table 4 below compares the performance of the present feature selection approach with other state of the art approaches.

Table 4: Predictive modeling results for RA dataset. In bold - best R2 and Spearman result. Multi-stage feature selection results depict only the best metric obtained out of the set of proxy models created. Full performance results of other proxy models are shown in supporting results.

| Features selector | Spearman Test (6)* | | Lasso (38) | | RFE (191) | | AIC (93) | | Multi-stage feature selection (34) | |
|---|---|---|---|---|---|---|---|---|---|---|
| Regressor | Test R2 | Test Spearman | Test R2 | Test Spearman | Test R2 | Test Spearman | Test R2 | Test Spearman | Test R2 | Test Spearman |
| Linear Regression | 0.42 | 0.71 | 0.45 | 0.72 | 0.33 | 0.65 | 0.37 | 0.68 | 0.39 | 0.67 |
| Random Forest | 0.55 | 0.74 | 0.58 | 0.77 | 0.57 | 0.76 | 0.50 | 0.71 | 0.57 | 0.75 |
| Gradient Boosting Regression | 0.55 | 0.74 | 0.56 | 0.75 | 0.56 | 0.76 | 0.51 | 0.71 | 0.57 | 0.76 |
| SVM | 0.41 | 0.70 | 0.45 | 0.72 | 0.43 | 0.70 | 0.42 | 0.69 | 0.41 | 0.67 |
| EBM | 0.53 | 0.73 | 0.53 | 0.74 | 0.51 | 0.73 | 0.47 | 0.71 | 0.54 | 0.75 |
| * The number in parentheses indicates the number of selected features for each method. | | | | | | | | | | |

With the exception of the AIC method, all other feature selection methods selected both CRP and ESR as features. This agrees well with their use in practice to determine DAS-28-CRP and DAS-28-ESR scores. Both markers are indicative of systemic inflammation and are used for diagnosis and monitoring of inflammatory conditions. As for additional features, the present feature selection approach identified cholesterol level as a feature, whereas the lasso algorithm did not select this. Lipid levels are known to be linked to RA activity and total cholesterol can be used for inflammation scoring. Another relevant feature, medical history of cardiac disorders, was also also uniquely selected by the present feature selection approach. This agrees with recent findings regarding the link between cardiovascular diseases and RA.

The global feature importances from the "Multiple strategy" RA proxy model were evaluated, and it was observed that CRP and ESR accounted for more than 20% of the predictive power. Other features like low-density lipoprotein (LDL), phosphates and LDH were found to be important to model predictions. The dependency plots between CRP and ESR and the DAS28-CRP was also analysed using single-feature proxy models. As CRP is used in calculation of DAS28-CRP, a strong positive association between them can be seen. Finally, there is a general trend indicating that higher ESR values correspond to larger disease severity, although this relationship is not as apparent

Other Endpoints

It will be understood that although RA and AD are discussed above, techniques described in this specification may also be used to select features and develop models to predict disease severity scores based on the endpoints of other clinical trials. For example, the claimed approach may also be used to select features and develop models to predict disease severity scores for other inflammatory conditions, for example a disease severity score for inflammatory bowel disease (IBD), for example a severity score for ulcerative colitis such as a MAYO score. By applying the present approach to a dataset for ulcerative colitis, it was found that model features may comprise at least one, or at least two, or all, of the following features: blood urea nitrogen, hemoglobin, platelets, age, albumin, alkaline phosphatase, calcium, chloride, creatinine, direct bilirubin, glucose, hematocrit, potassium, red blood cells, sodium, white blood cells, urine red blood cells, urine white blood cells.

General methodology

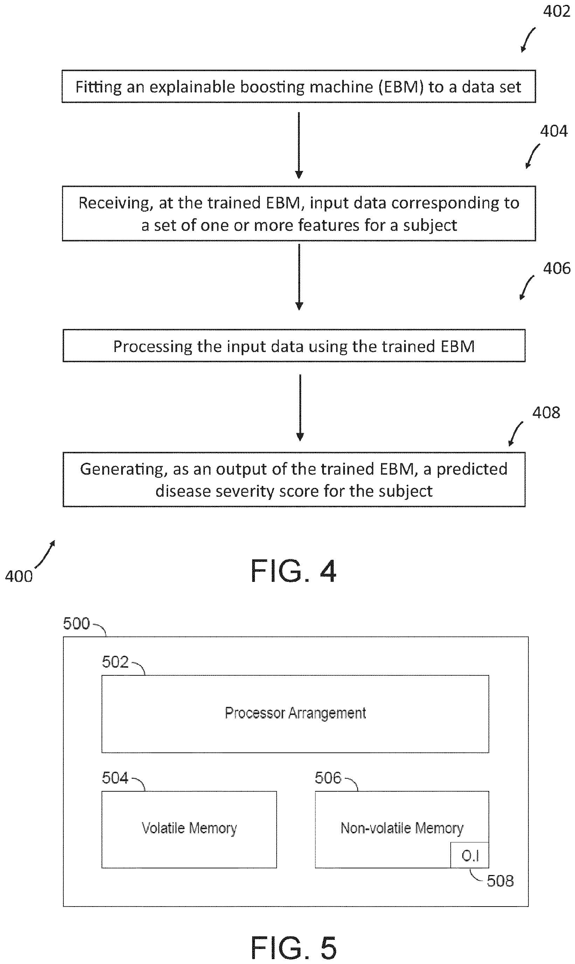

Figure 4 is a flow diagram of an example process 400 whereby an explainable boosting machine (EBM) is used to predict a disease severity score for a subject. For convenience, the process 400 will be described as being performed by a data processing system comprising one or more computers located at one or more locations.

As shown in Figure 4, an explainable boosting machine model is fitted to a data set (step 402), thereby providing a trained EBM. The training data set may be extracted from RCT data as described above.

To deploy the trained model, the system provides the trained EBM with input data corresponding to a set of one or more features of the subject (step 404), each feature corresponding to a model feature of the EBM. The input data may be extracted from RWD as described above.

The system processes the input data using the trained EBM (step 406), and generates, as an output of the EBM, a predicted disease severity score for the subject (step 408).

Figure 5 shows a schematic example of a system/apparatus which may be used for performing methods described herein. The system/apparatus shown is an example of a computing device. It will be appreciated by the skilled person that other types of computing devices/systems may alternatively be used to implement the methods described herein, such as a distributed computing system.

The apparatus (or system) 500 comprises one or more processors 502. The one or more processors control operation of other components of the system/apparatus 500. The one or more processors 502 may, for example, comprise a general purpose processor. The one or more processors 502 may be a single core device or a multiple core device. The one or more processors 502 may comprise a central processing unit (CPU) or a graphical processing unit (GPU). Alternatively, the one or more processors 502 may comprise specialised processing hardware, for instance a RISC processor or programmable hardware with embedded firmware. Multiple processors may be included.

The system/apparatus comprises a working or volatile memory 504. The one or more processors may access the volatile memory 504 in order to process data and may control the storage of data in memory. The volatile memory 504 may comprise RAM of any type, for example Static RAM (SRAM), Dynamic RAM (DRAM), or it may comprise Flash memory, such as an SD-Card.

The system/apparatus comprises a non-volatile memory 506. The non-volatile memory 506 stores a set of operation instructions 508 for controlling the operation of the processors 502 in the form of computer readable instructions. The non-volatile memory 506 may be a memory of any kind such as a Read Only Memory (ROM), a Flash memory or a magnetic drive memory.

The one or more processors 502 are configured to execute operating instructions 508 to cause the system/-apparatus to perform any of the methods described herein. The operating instructions 508 may comprise code (i.e. drivers) relating to the hardware components of the system/apparatus 500, as well as code relating to the basic operation of the system/apparatus 500. Generally speaking, the one or more processors 502 execute one or more instructions of the operating instructions 508, which are stored permanently or semi-permanently in the non-volatile memory 506, using the volatile memory 504 to temporarily store data generated during execution of said operating instructions 508.

Implementations of the methods described herein may be realised as in digital electronic circuitry, integrated circuitry, specially designed ASICs (application specific integrated circuits), computer hardware, firmware, software, and/or combinations thereof. These may include computer program products (such as software stored on e.g. magnetic discs, optical disks, memory, Programmable Logic Devices) comprising computer readable instructions that, when executed by a computer, such as that described in relation to Figure 5, cause the computer to perform one or more of the methods described herein.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An "active pharmaceutical ingredient" ("API"), or "active substance", in the broadest terms, refer to a molecule that has a biological or pharmacological effect on humans or animals. "API" or "active substances" include inter alia, but without limitation, biologies (e.g., antibodies, antibody-drug conjugates, small peptides, and the like) and small molecules (e.g., small molecular weight chemical compounds).In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (anti-diabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin. Examples of insulin derivatives are, for example, B29-N-myristoyl-des(B30) human insulin, Lys(B29) (N- tetradecanoyl)-des(B30) human insulin (insulin detemir, Levemir®); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl-ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba®); B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin.

Examples of GLP-1, GLP-1 analogues and GLP-1 receptor agonists are, for example, Lixisenatide (Lyxumia®), Exenatide (Exendin-4, Byetta®, Bydureon®, a 39 amino acid peptide which is produced by the salivary glands of the Gila monster), Liraglutide (Victoza®), Semaglutide, Taspoglutide, Albiglutide (Syncria®), Dulaglutide (Trulicity®), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro®), a cholesterol-reducing antisense therapeutic for the treatment of familial hypercholesterolemia or RG012 for the treatment of Alport syndrom.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc®), a sodium hyaluronate.

The term "antibody", as used herein, refers to an immunoglobulin molecule typically comprising four polypeptide chains (two heavy [H] chains and two light [L] chains inter-connected by disulfide bonds, i.e., a "full-length antibody"), as well as multimers thereof (e.g., IgM), and antigen-binding portions or fragments thereof. Antigen-binding portions or fragments of antibodies can comprise a cleaved portion of a full-length antibody, although the term is not limited to such cleaved fragments. Examples of antigen-binding portions or fragments of immunoglobulin molecules include F(ab)and F(ab')2 fragments, as well as scFv (single-chain Fv), di-scFv, tri-scFv, etc., which all retain the ability to bind their antigen. Other engineered molecules, such as domain specific antibodies, single domain antibodies, domain-deleted antibodies, chimeric antibodies, CDR-grafted antibodies, diabodies, triabodies, tetrabodies, minibodies, intrabodies, immunoglobulin single variable domains (e.g., VHHs), small modular immunopharmaceuticals (SMIPs), and shark variable IgNAR domains, are also encompassed within the expression "antigen-binding portions or fragments". Additional examples of antigen-binding fragments are known in the art. The antibody or the antigen-binding portion or fragment thereof can be polyclonal, monoclonal, recombinant, chimeric, de-immunized, or humanized, fully human, non-human (e.g., murine). In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes multispecific antigen-binding molecules, such as bispecific, tri-specific, tetraspecific, etc. antibodies or antigen-binding fragments thereof. A multispecific antigen-binding molecule may be monovalent or multivalent (e.g., bivalent, trivalent, tetravalent, etc.). For instance, a multispecific antigen-binding molecule may be specific for two or more different epitopes of a same antigen, or may contain antigen-binding domains specific for epitopes of more than one antigen. Non-limiting examples include tetravalent bispecific tandem immunoglobulins (TBTI) and dual variable region antibody-like binding proteins having cross-over binding region orientation (CODV).

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 receptor (IL-6R) mAb (e.g., Sarilumab), and anti IL-4 receptor (IL-4R) mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope and spirit of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

Many modifications and variations to the embodiments described herein will be evident to those skilled in the art, which fall within the definition of the following claims:

**Claims**

1. A computer-implemented method of predicting disease severity in a subject, comprising:

receiving, at a generalized additive model, input data corresponding to a set of one or more features for the subject, and

generating, as an output of the generalized additive model, a predicted disease severity score for the subject, wherein generating the predicted disease severity score for the subject comprises processing the received input data using the generalized additive model.

2. The computer-implemented method of claim 1, wherein the generalized additive model comprises an explainable boosting machine.

3. The computer-implemented method of claim 1 or claim 2, wherein the disease severity score is a disease severity score for an inflammatory condition.

4. The computer-implemented of any one of the preceding claims, wherein the set of one or more features comprise one or more physiological measurements

5. The computer-implemented method of any one of the preceding claims, wherein the disease severity score is a rheumatoid arthritis disease activity score such as a DAS-28 score.

6. The computer-implemented method of claim 5, wherein the set of one or more features comprise one or both of:

an erythrocyte sedimentation rate, ESR, measurement for the subject, or
a C-reactive protein, CRP, measurement for the subject.

7. The computer-implemented method of claim 5 or 6, wherein the set of one or more features comprise at least one of:

a low-density lipoprotein, LDL, measurement for the subject;
a phosphates measurement for the subject,
a lactate dehydrogenase, LDH, measurement for the subject,
a cholesterol (CHOL) measurement for the subject,
a high-density lipoprotein, HDL, measurement for the subject,
a blood urea nitrogen, BUN, measurement for the subject,
an indirect bilirubin, IB, measurement for the subject, or
a urate measurement for the subject.

8. The computer-implemented method of any one of claims 1 to 4, wherein the disease severity score is a severity score for atopic dermatitis, such as an EASI score.

9. The computer-implemented method of claim 8, wherein the set of one or more features comprise at least one of:

a lactase dehydrogenase, LDH, measurement, for the subject;
a C-reactive protein, CRP, measurement for the subject;
an allergen-specific immunoglobulin, ALTIGE, measurement for the subject;
an eosinophils, EOS, measurement for the subject;
a lymphocytes, LYM, measurement for the subject;
a creatine kinase, CK, measurement for the subject;
a basophils, BASO, measurement for the subject;
an aspartate transferase, AST, measurement for the subject, or
an albumin, ALB, measurement for the subject.

10. The computer-implemented method of claim 9, wherein the set of one or more features comprises:

LDH and CRP,
LDH and ALTIGE,
ALTIGE and EOS, or
EOS and LYM.

11. The computer-implemented method of any one of claims 1 to 4, wherein the disease severity score is a severity score for inflammatory bowel disease, such as a severity score for ulcerative colitis such as a MAYO score.

12. The computer-implemented method of claim 11, wherein the set of one or more features comprise at least one of:

a blood urea nitrogen measurement for the subject;
a hemoglobin measurement for the subject;
a platelets measurement for the subject;
an albumin measurement for the subject;
an alkaline phosphatase measurement for the subject;
a calcium measurement for the subject;
a chloride measurement for the subject;
a creatinine measurement for the subject;
a direct bilirubin measurement for the subject;
a glucose measurement for the subject;
a hematocrit measurement for the subject;
a potassium measurement for the subject;
a red blood cells measurement for the subject;
a sodium measurement for the subject;
a white blood cells measurement for the subject;
a urine red blood cells measurement for the subject, or
a urine white blood cells measurement for the subject.

13. The computer-implemented method of claim 11 or claim 12, wherein the set of one or more features comprise at least two, or all, of:

a blood urea nitrogen measurement for the subject;
a hemoglobin measurement for the subject;
a platelets measurement for the subject;
an albumin measurement for the subject;
an alkaline phosphatase measurement for the subject;
a calcium measurement for the subject;
a chloride measurement for the subject;
a creatinine measurement for the subject;
a direct bilirubin measurement for the subject;
, a glucose measurement for the subject;
a hematocrit measurement for the subject;
a potassium measurement for the subject;
a red blood cells measurement for the subject;
a sodium measurement for the subject;
a white blood cells measurement for the subject;
a urine red blood cells measurement for the subject, or
a urine white blood cells measurement for the subject.

14. The computer-implemented method of any one of the preceding claims, further comprising augmenting a patient data set using the generalized additive model, wherein augmenting the patient data set comprises deploying the generalized additive model on a patient data set to predict, for each of a plurality of patients, a predicted disease severity score for the patient.

15. The computer-implemented method of claim 14, wherein the patient data set is a set of real world data.

16. A computer-implemented method of determining a response of a subject to a drug over time, comprising, for each of a plurality of times in a time series, generating a disease severity score for the subject in accordance with the method of any one of the preceding claims, wherein the disease severity score for the subject at each time is generated based on values of at least one of said set of one or more features at that time.

17. A computer-implemented method of providing a trained generalized additive model to predict a disease severity score for a subject, the method fitting the generalized additive model to a data set comprising a plurality of data items for a respective plurality of subjects, each data item comprising a disease severity score for the subject and data corresponding to a set of one or more features for the subject.

18. The computer-implemented method of claim 17, wherein the trained generalized additive model comprises an explainable boosting machine.

19. A non-transitory computer-readable medium comprising instructions, which when executed by a processor, cause the processor to perform the method of any one of the preceding claims.

20. A computing system comprising one or more processors and one or more memories storing computer readable instructions, which when executed by the one or more processors, cause the method of any one of claims 1 to 18 to be performed.

21. A method of treating an immune-mediated disease in a subject in need thereof, comprising:

   • predicting a disease severity score for the subject, that includes:

      receiving, at a generalized additive model, input data corresponding to a set of one or more features for the subject, and
      generating, as an output of the generalized additive model, the predicted disease severity score for the subject by processing the received input data using the generalized additive model, and

   • administering an active substance based on the predicted disease severity score.

22. The method of claim 21, wherein the subject has been diagnosed as being affected with the immune-mediated disease based on the predicted disease score.

23. The method of claim 21 or 22, wherein the immune-mediated disease is atopic dermatitis.

24. The method of claim 23, wherein the active substance is an anti-interleukin-4-receptor (IL-4R) antibody or an antigen-binding fragment thereof.

25. The method of claim 24, , wherein the anti-IL-4R antibody or antigen binding fragment thereof is administered in a dosing regimen based on the predicted disease severity score, and the dosing regimen comprises an initial dose followed by one or more secondary doses, wherein the initial dose is about 600 mg of the anti-IL-4R antibody or antigen-binding fragment thereof, the secondary dose is about 300 mg of the anti-IL-4R antibody or antigen-binding fragment thereof, and each secondary dose is administered 1 to 2 weeks after the immediately preceding dose.

26. The method of claim 21 or 22, wherein the immune-mediated disease is rheumatoid arthritis.

27. The method of claim 26, wherein the active substance is an anti-interleukin-6-receptor (IL-6R) antibody or an antigen-binding fragment thereof.

28. The method of claim 27, wherein the anti-IL-6R antibody or antigen binding fragment thereof is administered in a dosing regimen based on the predicted disease severity score and the dosing regimen comprises one or more doses of from about 150 mg to about 200 mg of the anti-IL-6R antibody or antigen-binding fragment thereof administered once about every two weeks.

29. The method of claim 21 or 22, wherein the immune-mediated disease is juvenile idiopathic arthritis (JIA), preferably polyarticular-course JIA (pcJIA) or systemic JIA (sJIA), more preferably pcJIA.

30. The method of claim 29, wherein the active substance is an anti-interleukin-6-receptor (IL-6R) antibody or an antigen-binding fragment thereof to the subject.

31. The method of claim 30, wherein the anti-IL-6R antibody or antigen binding fragment thereof is administered in a dosing regimen based on the predicted disease severity score, and the dosing regimen comprises one or more doses of from about 2 mg/kg to about 4 mg/kg of the anti-IL-6R antibody or antigen-binding fragment thereof administered once about every two weeks.

32. The method according to claim 31, wherein:

- the subject's body weight is greater than or equal to 10 kg and less than 30 kg and the dosing regimen comprises one or more doses of about 4 mg/kg of the anti-IL-6R antibody or antigen-binding fragment thereof administered once every two weeks; or

- the subject's body weight is greater than or equal to 30 kg and the dosing regimen comprises one or more doses of about 3 mg/kg of the anti-IL-6R antibody or antigen-binding fragment thereof administered once every two weeks, and each one or more doses of the anti-IL-6R antibody or antigen-binding fragment thereof is capped at 200 mg.

33. An active substance, for use in a method of treating an immune-mediated disease as defined in any one of claims 21 to 32.

34. An anti-interleukin-4-receptor (IL-4R) antibody or an antigen-binding fragment thereof, for use in the method of treating atopic dermatitis as defined in any one of claims 23 to 25.

35. An anti-interleukin-6-receptor (IL-6R) antibody or an antigen-binding fragment thereof, for use in a method of treating rheumatoid arthritis as defined in any one of claims 26 to 28.

36. An anti-interleukin-6-receptor (IL-6R) antibody or an antigen-binding fragment thereof, for use in a method of treating juvenile idiopathic arthritis (JIA), preferably polyarticular-course JIA (pcJIA) or systemic JIA (sJIA), more preferably pcJIA, as defined in any one of claims 29 to 32.

FIG. 1

B. Multi-stage Feature Selection

RCT
CDM

Free-pass list

Missing drop out — 201

Data-type selector — 202

Variance threshold — 203

204

Mutual info

Select from model

f_regression

Feature shortlist

RWD availability check

RWD

205

Proxy model feature candidates

**FIG. 2**

300

a

b

d

**FIG. 3**

402

Fitting an explainable boosting machine (EBM) to a data set

404

Receiving, at the trained EBM, input data corresponding to a set of one or more features for a subject

406

Processing the input data using the trained EBM

408

Generating, as an output of the trained EBM, a predicted disease severity score for the subject

400

# FIG. 4

500

502

Processor Arrangement

504

Volatile Memory

506

Non-volatile Memory

O.I

508

# FIG. 5

EUROPEAN SEARCH REPORT

Application Number

EP 24 31 5027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG HAOLIN ET AL: "Integrating Co-Clustering and Interpretable Machine Learning for the Prediction of Intravenous Immunoglobulin Resistance in Kawasaki Disease",<br>IEEE ACCESS, IEEE, USA,<br>vol. 8, 20 May 2020 (2020-05-20), pages 97064-97071, XP011791295,<br>DOI: 10.1109/ACCESS.2020.2996302<br>[retrieved on 2020-06-01]<br>* the whole document *<br>----- | 1-36 | INV.<br>G16H50/20<br>G06N3/00<br>G16H50/30 |
| T | Moriarty Kathleen P ET AL: "Proxy Endpoints - Bridging Clinical Trials and Real World Data",<br>,<br>19 April 2024 (2024-04-19), XP093171960,<br>DOI: 10.2139/ssrn.4784962<br>Retrieved from the Internet:<br>URL:https://ssrn.com/abstract=4784962<br>* the whole document *<br>----- | | |
| | -/-- | | |

**TECHNICAL FIELDS SEARCHED (IPC)**

G16H
G06N

The present search report has been drawn up for all claims

| Place of search | Date of completion of the search | Examiner |
|---|---|---|
| Munich | 20 September 2024 | Heidrich, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 24 31 5027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | THOMAS BJORN R. ET AL: "Modeling of Temporal Exposure to the Ambient Environment and Eczema Severity", JID INNOVATIONS, vol. 2, no. 1, 1 January 2022 (2022-01-01), page 100062, XP093200644, ISSN: 2667-0267, DOI: 10.1016/j.xjidi.2021.100062 Retrieved from the Internet: URL:https://pdf.sciencedirectassets.com/77 8506/1-s2.0-S2667026721X00069/1-s2.0-S2667 026721000631/main.pdf?X-Amz-Security-Token =IQoJb3JpZ2luX2VjEIj//////////wEaCXVzLWVhc 3QtMSJHMEUCIBj0cycu7y0Wdhxj22bOs0SaoduiHfO SNXUuFM4a7VgdAiEA3QvsxaFu4X6+5GbLyeypoBYba s3yRzJuxqQq194cKvAqvAUIof//////////ARAFGgw wNTkwMDM1N> * abstract * - - - - - | 8-10, 23-25,34 | |
| A | OTTO-SOBOTKA FABIAN ET AL: "Modeling determinants of satisfaction with health care in youth with inflammatory bowel disease part 2: semiparametric distributional regression", CLINICAL EPIDEMIOLOGY, vol. Volume 11, 1 May 2019 (2019-05-01), pages 403-417, XP093200653, ISSN: 1179-1349, DOI: 10.2147/CLEP.S191458 Retrieved from the Internet: URL:https://www.dovepress.com/getfile.php? fileID=49915> * abstract; figure 1 * - - - - - -/-- | 11-13 | TECHNICAL FIELDS SEARCHED (IPC) |

The present search report has been drawn up for all claims

| Place of search | Date of completion of the search | Examiner |
|---|---|---|
| Munich | 20 September 2024 | Heidrich, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

**Application Number**

EP 24 31 5027

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MCKIRDY SHONA ET AL: "Handgrip strength as a surrogate marker of lean mass and risk of malnutrition in paediatric patients", CLINICAL NUTRITION, vol. 40, no. 9, 1 September 2021 (2021-09-01), pages 5189-5195, XP093200671, GB ISSN: 0261-5614, DOI: 10.1016/j.clnu.2021.08.005 Retrieved from the Internet: URL:https://pdf.sciencedirectassets.com/272493/1-s2.0-S0261561421X0009X/1-s2.0-S0261561421003769/main.pdf?X-Amz-Security-Token=IQoJb3JpZ2luX2VjEIn//////////wEaCXVzLWVhc3QtMSJHMEUCIQC5QhvDER9uelwtlrTzpulnqdoITHj1rUZ4aF/NU04MRwIgYrJUs/sUioyI5xrVcGkh0LfnQpNpgLA1i9ful5UuGe8qvAUIov//////////ARAFGgwwNTkwMDM1N> * abstract, section "I. Introduction" * | 29 | |
| A | RASHED AHMED MOHAMMED ET AL: "Association of hand grip strength with disease activity, disability and quality of life in children and adolescents with Juvenile Idiopathic Arthritis", ADVANCES IN RHEUMATOLOGY, vol. 58, no. 1, 1 December 2018 (2018-12-01), XP093200665, ISSN: 2523-3106, DOI: 10.1186/s42358-018-0012-1 Retrieved from the Internet: URL:https://advancesinrheumatology.biomedcentral.com//advancesinrheumatology.biomedcentral.com/counter/pdf/10.1186/s42358-018-0012-1.pdf> * abstract * | 29 | **TECHNICAL FIELDS SEARCHED (IPC)** |

- / - -

The present search report has been drawn up for all claims

| Place of search | Date of completion of the search | Examiner |
|---|---|---|
| Munich | 20 September 2024 | Heidrich, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 4

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/160465 A1 (SANOFI BIOTECHNOLOGY [FR]; REGENERON PHARMA [US] ET AL.) 6 August 2020 (2020-08-06) * claim 1 * | 30-32,36 | |

----- 

TECHNICAL FIELDS SEARCHED (IPC)

The present search report has been drawn up for all claims

| Place of search | Date of completion of the search | Examiner |
|---|---|---|
| Munich | 20 September 2024 | Heidrich, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................
& : member of the same patent family, corresponding document

EPO FORM 1503 03.82 (P04C01)

page 4 of 4

Europäisches
Patentamt

European
Patent Office

Office européen
des brevets

**Application Number**

EP 24 31 5027

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patent application does not comply with the
requirements of unity of invention and relates to several inventions or groups of inventions, namely:

1. claims: 1-36

   Diagnosing and treating rheumatoid arthritis, atopic
   dermatitis, IBD, juvenile idiopathic arthritis

1.1. claims: 1-7, 14-22, 26-28, 33, 35

   Diagnosing and treating rheumatoid arthritis

1.2. claims: 1-4, 8-10, 14-25, 33, 34

   Diagnosing and treating atopic dermatitis

1.3. claims: 1-4, 11-22

   Diagnosing IBD

1.4. claims: 21, 22, 29-33, 36

   Diagnosing and treating juvenile idiopathic arthritis
   - - -

Please note that all inventions mentioned under item 1, although not
necessarily linked by a common inventive concept, could be searched
without effort justifying an additional fee.

## ANNEX TO THE EUROPEAN SEARCH REPORT
## ON EUROPEAN PATENT APPLICATION NO.

EP 24 31 5027

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2024

| Patent document cited in search report | Publication date | Patent family member(s) | Publication date |
|---|---|---|---|
| WO 2020160465 A1 | 06-08-2020 | AU 2020216978 A1 | 09-09-2021 |
| | | BR 112021014377 A2 | 28-12-2021 |
| | | CA 3128212 A1 | 06-08-2020 |
| | | CN 114206442 A | 18-03-2022 |
| | | CO 2021011352 A2 | 10-12-2021 |
| | | EP 3917618 A1 | 08-12-2021 |
| | | IL 284890 A | 31-08-2021 |
| | | JP 7542543 B2 | 30-08-2024 |
| | | JP 2022519828 A | 25-03-2022 |
| | | KR 20210122810 A | 12-10-2021 |
| | | MA 54861 A | 08-12-2021 |
| | | SG 11202107735S A | 30-08-2021 |
| | | TW 202043285 A | 01-12-2020 |
| | | US 2020339693 A1 | 29-10-2020 |
| | | US 2023127528 A1 | 27-04-2023 |
| | | WO 2020160465 A1 | 06-08-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HARSHA NORI** ; **SAMUEL JENKINS** ; **PAUL KOCH** ; **RICH CARUANA**. Interpretml: A unified framework for machine learning interpretability. *ar-Xiv:1909.09223*, 2019